# EUROPEAN PATENT APPLICATION

(11) **EP 1 640 020 A1**
(43) Date of publication of application: **29.03.2006**
(21) Application number: 04732417.3
(22) Date of filing: 12.05.2004
(51) Int. Cl.: A61K 45/00, A61P 9/04, A61P 9/06

(54) **CARDIOPROTECTIVE AGENT**

(30) Priority: 13.05.2003 JP 2003134487
(71) Applicant: Miyazaki, Mizuo, Nagaokakyo-shi, Kyoto 617-0843 (JP)
(72) Inventor: MIYAZAKI, Mizuo, Nagaokakyo-shi, Kyoto 6170843 (JP); TAKAI, Shinji, Takatsuki-shi, Osaka 5691123 (JP)
(74) Representative: De Clercq, Ann G. Y.
(86) International application number: PCT/JP2004/006384
(87) International publication number: WO 2004/100988

(57) **Abstract**

A problem of the present invention is to provide an agent which allows effective protection of cardiac damage in a case where a variety of symptoms such as arrhythmia, cardiac desmoplasia and heart-failure are likely to accompany with hypertension, hypercardia, myocardial infarction, arteriosclerosis, diabetic and non-diabetic renal diseases, and re-stenosis posterior to PTCA. The agent contains an effective amount of at least one protease inhibitor to administer intravenously or orally. The protease inhibitor is preferably a serine protease inhibitor. The serine protease inhibitor is preferably a chymotrypsin-like serine protease inhibitor. Concretely, it is a chymase inhibitor such as a peptide derivative of aryl diester of alpha-aminoalkylphosphonic acid which is Suc-Val-Pro-Phe^{p} (OPh)₂, and preferably the enantiormer Suc-Val-Pro-L-Phe^{p} (OPh)₂.

## Description

### Technical Field of the Invention

The present application claims the priority based on Japanese Patent Application No. 2003-134487 which is incorporated herein for reference.
The present invention relates to a protease inhibitor having an action for protecting cardiac damage. More specifically, this invention relates to a serine protease inhibitor, particularly a chymase inhibitor, which has an action for protecting cardiac damage after the treatment of myocardial infarction or angina pectoris.

### Background of the Invention

Conventionally, treatments of myocardial infarction or angina pectoris have been classified into medical internal and surgical treatment methods to carry out several procedures. At present, the medical internal treatments used in the clinical field are mainly thrombolytic treatments, wherein fibrinolytic drugs such as a tissue plasminogen activator (t-PA) and urokinase (UK) are used to remove a thrombus which may cause myocardial infarction or the like. However, the fibrinolytic drugs can not be exempt from a problem that they would be administered excessively to bring about lysis of even a thrombus essential for the living organism in the other tissues than the target, resulting in a serious complicated side effect such as intracerebral hemorrhage. Moreover, these fibrinolytic drugs, which decompose fibrin to attain thrombolysis, can not bring under a specific condition a sufficient effect on a thrombus that contains no fibrin-derived substance.

Therefore, at present, the surgical treatments described below are carried out in preference to the medical internal treatments. Nowadays, as a surgical treatment for canceling the occlusion and stenosis of the blood vessel which may cause myocardial infarction and angina pectoris, percutaneous transluminal coronary angioplasty (PCTA), coronary artery bypass graft (CABG) and the like are carried out. However, these methods, which expand the section or make the detour of an occluded or constricted blood vessel as a treatment for the main coronary artery from the viewpoint of actual situation of the treatment, even if done successfully, can not completely dissolve ischemia in a cardiac muscle with the ischemia yet left locally, resulting in no sufficiently recovered cardiac functions.

On the other hand, a variety of factors having a vascularization action have been attempted to use for the above object, that is, allowing angiogenesis in the ischemic site of a cardiac muscle and promoting development of collateral vessels, thereby to supply the ischemic cardiac muscle with fresh blood. Moreover, similarly in the other surgical treatment, a new trial has been carried out. Specifically, it is a treatment by transmyocardinal laser revascularization (TMLR or TMR) using laser (Cardiovasc. Today, 1, p.939-946, 1997). This TMLR is a treatment method wherein lasering in a cardiac muscle makes a channel, through which the cardiac muscle is directly perfused with blood from the ventricle. This method is reported to be effective for treatment of coronary stenosis (Horvath KA et al., J. Thoracic and Cardiovasc Surg., 111 , p. 1041-1053, 1996, Cooley DA et al., J. Thoracic and Cardiovasc Surg., 111, p. 791-799, 1996). However, this method has problems that the channel is clogged. The follow-up study gives a result that it is not so effective (Burkhoff D, Ann Thrac Surg., 61, p. 1532-1535, 1996).

Then, recently, the improved TMLR method (wherein lasering around a coronary artery makes a channel to promote vascularization) has been developed. Furthermore, VEGF, an angiogenesis factor was used to avoid the channel from being clogged in order to improve the method. However, the combination of VEGF with the improved TMLR method gave no acknowledged result for therapy (Annals of Thoracic Surgery, 62, p. 1051-1058, 1996). As described above, the medical internal therapies or surgical therapies of myocardial infarction or angina pectoris have been known, but their concrete effectiveness remain to clarified.

It is considered that Angiotensin II (Ang II) has a cell proliferation promotion action in addition to a vasopressive action, and so is a causative substance or a risk factor for diseases such as hypertension, hypercardia, myocardial infarction, arteriosclerosis, diabetic and non-diabetic renal diseases, and re-stenosis posterior to PTCA. Moreover, it is known that angiotensin conversion enzyme (ACE) acts to convert Angiotensin I (AngI) to AngII, and hence many ACE inhibitors have been developed as agents for preventing and treating the above-described diseases. However, the results of MERCAPTOR test (Circulation, 86, 1, p. 100, 1992) and MARCAPTOR test (J. Am. Coll. Cardiol., 27, 1, p. 1 1996), which were carried out to expect the prevention effect of an ACE inhibitor against the re-stenosis posterior to PTCA, gave no demonstrated effectiveness. The results of the above clinical trial suggested that human has an AngII production pathway without ACE involved in. On the other hand, Okunishi et al have clarified that the enzyme called as chymase, a kind of serine protease, converts more highly and selectively AngI toAngII than ACE (Biochem. Biophys. Res. Commun., 149, p. 1186. 1987). Then, it is desired that a clinically applicable chymase inhibitor is developed to result in the prevention and treatment of diseases in a cardiac /circulatory system caused by the abnormal sthenia in the production of AngII. In order to solve the above-described problem, an invention has been disclosed to use a chymase inhibitor as an agent for preventing and treating the diseases in the cardiac / circulatory system (Patent documents 1, 2).

However, the above-described chymase inhibitor is not clarified in manner, dose and the like of the administration, and it is not clear whether or not it sufficiently exerts the effect.
[Patent document 1] Japanese Patent Application Laid-open No. JP2000-95770, A
[Patent document 2] Japanese Patent Application Laid-open No. JP10-53579, A (1998)

### [Disclosure of the Invention]

### [Problem to be solved by the Invention]

An object of the prevent invention is to provide an agent which relaxes symptoms such as arrhythmia, cardiac desmoplasia, and heart-failure in a case where the symptoms are likely to accompany with hypertension, hypercardia, myocardial infarction, vasculosclerosis, diabetic and non-diabetic renal diseases, and re-stenosis posterior to PTCA, allowing effective protection of cardiac damage.

### [Means for Solving the Problem]

The present inventors have taken note of the fact that in case concerned about the symptoms of heart-failure an efficient amount of at least one protease inhibitor is administered in a sufficient dose for protecting cardiac damage for a certain period, allowing improvement of symptoms accompanying with the above-described diseases, and made a diligent study. As a result, they have found that an agent containing the relevant protease inhibitor can be administered intravenously or orally to solve the above-described object. Thus, the present invention has been completed.

Specifically, the present invention comprises,
1. An agent for protecting cardiac damage, wherein the agent contains an effective amount of at least one protease inhibitor and is administered intravenously or orally,
2. The agent for protecting cardiac damage according to Item 1, wherein the protease inhibitor is a serine protease inhibitor,
3. The agent for protecting cardiac damage according to Item 2, wherein the serine protease inhibitor is a chymotrypsin-like serine protease inhibitor,
4. The agent for protecting cardiac damage according to Item 3, wherein the chymotrypsin-like serine protease inhibitor is a chymase inhibitor,
5. The agent for protecting cardiac damage according to Item 4, wherein the chymase inhibitor is a peptide derivative of aryl diester of alpha- aminoalkylphosphonic acid,
6. The agent for protecting cardiac damage according to Item 4, wherein the chymase inhibitor is Suc-Val-Pro-Phe^{p}(OPh)₂,
7. The agent for protecting cardiac damage according to Item 4, wherein the chymase inhibitor is an enriched preparation of enantiomer Suc-Val-Pro-L-Phe^{p} (OPh)₂ of Suc-Val-Pro-Phe^{p} (OPh)₂,
8. The agent for protecting cardiac damage according to Item 7, wherein the Suc-Val-Pro-L-Phe^{p}(OPh)₂ comprises greater than 95% by weight of the total Suc-Val-Pro-Phe^{p}(OPh)₂ in the enriched preparation of the enantiomer,
9. The agent for protecting cardiac damage according to any one of Items 1-8, wherein the protease inhibitor is bound to a transmitter for maintaining an effective local concentration of the protease inhibitor in the relevant site and then administered, the transmitter being a carrier having a high molecular weight selected from the group consisting of hyaluronic acid, hydrogel, carboxymethylcellose, dextran, cyclodextran and a composition of compounds thereof,
10. An agent mixture for protecting cardiac damage, comprising the protease inhibitor according to any one of Items 1-9 and a pharmaceutically acceptable diluent solution or excipient,
11. A method for improving arrhythmia, cardiac desmoplasia and / or heart-failure, wherein the agent mixture for protecting cardiac damage according to Item 10 is administered to a vertebrate subject in a case where the arrhythmia, cardiac desmoplasia, and heart-failure are likely to accompany with hypertension, hypercardia, myocardial infarction, arteriosclerosis, diabetic and non-diabetic renal diseases, re-stenosis posterior to PTCA,
12. A use of the agent mixture for protecting cardiac damage according to Item 10, wherein the use comprises making a medicine which is applied against arrhythmia, cardiac desmoplasia and / or heart-failure in a case where the arrhythmia, cardiac desmoplasia and / or heart-failure are likely to accompany with hypertension, hypercardia, myocardial infarction, arteriosclerosis, diabetic and non-diabetic renal diseases, and re-stenosis posterior to PTCA, and
13. A use of the agent mixture for protecting cardiac damage according to Item 10, wherein the use comprises using as an agent for improving arrhythmia, cardiac desmoplasia and / or heart-failure in a case where the arrhythmia, cardiac desmoplasia and / or heart-failure are likely to accompany with hypertension, hypercardia, myocardial infarction, arteriosclerosis, diabetic and non-diabetic renal diseases, and re-stenosis posterior to PTCA.

### [Effects of the Invention]

The present invention relates to the improvement of a variety of symptoms such as arrhythmia, cardiac desmoplasia, and heart-failure accompanying with hypertension, hypercardia, myocardial infarction, arteriosclerosis, diabetic and non-diabetic renal diseases, and re-stenosis posterior to PTCA of a vertebrate subject, wherein an agent containing an effective amount of a protease inhibitor is administered intravenously or orally to the subject. The relevant protease inhibitor can be administered, for example, during or after the treatment of these diseases.

### [Description of the preferred embodiment(s)]

The agent of the present invention is an agent for improving a variety of symptoms such as arrhythmia, cardiac desmoplasia, and heart-failure accompanying with hypertension, hypercardia, myocardial infarction, arteriosclerosis, diabetic and non-diabetic renal diseases, and re-stenosis posterior to PTCA of a warm blooded mammal, wherein an effective amount of at least one serine protease inhibitor is administered intravenously or orally to the mammal in a sufficient dose for protecting cardiac damage for a certain period. The preferred embodiment relates to a method for protecting cardiac damage using at least one serine protease inhibitor, a chymotrypsin-like serine protease inhibitor. The agent of the present invention can be applied to human as the warm blooded mammal.
In the present invention, the cardiac damage protecting action can be expected against arrhythmia, cardiac desmoplasia and heart-failure accompanying with hypertension, hypercardia, myocardial infarction, arteriosclerosis, diabetic and non-diabetic renal diseases, and re-stenosis posterior to PTCA.

### (Protease inhibitor)

The protease inhibitor contained in the agent of the present invention is a known substance. Any protease inhibitor can be used regardless of a method used for preparing it, as long as it is purified to a necessary degree for a pharmaceutical use.

One preferred group of proteases, as targeted by the agent for protecting cardiac damage of the present invention are the serine proteases. The serine proteases are the subclass of endopeptidase which cleaves and binds serine in a peptide (Barrett, A. J. , In: Protease Inhibitors, Ed. Barrett, A. J. et al., Elsevier, Amsterdam, pp.3-22, 1986). Serine protease itself is known. For example, two super families of serine proteases, i.e., a chymotrypsin super family and a *Streptomyces* subtilisin super family have been reported.

The serine protease inhibitors are known, and divided into the following families: including (1) a family of trypsin inhibitor (Kunitz) derived from pancreas of bovine, is also known as a basic protease inhibitor (Ketcham, L. K. et al, In: Atlas of Protein Sequence and Structure, Ed. Dayhoff, M. O., pp. 131-143, 1978) (hereinafter, abbreviated as "BPTI"), (2) the Kazal family, (3) the *Streptomyces* subtilisin inhibitor family (hereinafter, abbreviated as "SSI"), (4) the soybean trypsin inhibitor (Kunitz) family, (6) the potato inhibitor family, (7) Bowman-Birk family (Laskowski, M. et al, Ann. Rev. Biochem., 49: pp. 593-626, 1980). Crystallographic data are available for a number of intact inhibitors including members of the BPTI, Kazal, SSI, soybean trypsin, potato trypsin families and for a cleaved form of the Seprin-alpha-1-antitrypsin cleavage type (Read, R. J. et al., In: Protease inhibitors, Ed. Barret. A. J. et al.,: Elsevier, Amsterdam, p.301-336, 1986). Many serine protease inhibitors have specificity for a broad range of protease families, and can inhibit both the chymotrypsin super family of protease including blood coagulation serine protease and the Streptomyces subtilisin super family of serine protease (Laskowski et al., Ann. Rev. Biochem., 49: pp. 593-626, 1980). The specificity of each inhibitor is thought to be determined by the identity of an amino acid at the amino-terminal with respect to the direct cleavage site of serine protease. It is considered that this amino acid known as a P site residue forms an acyl bond with serine in the active site of the serine protease (Laskowski et al, Ann. Rev. Biochem., 49: pp. 593-626, 1980).

The preferred serine protease inhibitor contained in the agent of the present invention belongs to the serpin family and the Bowman-Birk family. The serine protease inhibitor against the serpin family includes plasminogen activation factor inhibitors PAI-1, PAI-2, PAI-3, and C1 esterase- inhibitor, alpha-2-anti-plasmin, contrapsin, alpha-1-anti-trypsin, anti-thrombin III, protease nexin I, alpha-1-anti-chymotrypsin, protein C inhibitor, heparin co-factor II and growth hormone adjusting protein are listed (Carrell et al, Cold Spring Harbor Symp. Quant. Biol. , 52: 527-535, 1987).

The example of serine protease of chymotrypsin super family includes tissue-type plasminogen activator: t-PA, trypsin, trypsin-like protease, chymotrypsin, plasmin, elastase, urokinase (or non-urine type plasminogen activation factor: u-PA) , acrosin, activated protein C, C1 esterase, cathepsin G, chymase, and , protease of blood coagulation cascade including kallikrein, thrombin, factor VIIa, factor IXa, factor Xa, factor XIa, factor XIIa and the like are included (Barrett, A. J., In: Protease Inhibitors, Ed. Barrett, A. J. et al., Elsevier, Amsterdam, p.3-22, 1986, Strassburger, W. et al. FEBS lett., 157: p.219-223, 1983).

The catalytic domain in all the serine protease of chymotrypsin super family has homology in sequence and homology in structure. The homology in sequence comprises (1) specific activation site residue, for example, in the case of trypsin, serine located at 195, histidine at 57, and aspartic acid at 102 which are common; (2) an oxyanion hole (for example, glycine at 193 and aspartic acid at 194 in the case of trypsin), (3) a cysteine residue forming a disulfide bridges in the structure, which are all reserved (Hartley, B. S., Symp. Soc. Gen. Micrbopl., 24: 152-182 (1974)).

The structural homology includes (1) the common fold consists of two Greek key structures (Richardson), (2) a common disposition of catalytic residue, (3) detailed conservation of the structure within the core of the molecule (Stroud, R. M., Sci. AM., 231: 24-88).

The agent of the present invention may contain the peptide derivative of an aryl diester of alpha-aminoalkylphosphonic acids. These alpha-aminoalkylphosphonate derivatives have been found to be the inhibitors of several serine proteases including bovine thrombin, human factor XIIa, human factor Xa, human kallikrein, bovine trypsin, rat skin tryptase, human leukocyte elastase, porcine pancreas elastase, bovine chymotrypsin, human leukocyte cathepsin G, rat mast cell protease II, (USP No.5,543,396; No.5,686,419; No.5,952,307; and the others). These derivatives are contained in human blood plasma, and remarkably stable under a variety of circumstances. For example, the preferred inhibitor Suc-Val-Pro-Phe^{p}(OPh)₂, particularly preferably, Suc-Val-Pro-L-Phe^{p}(OPh)₂ contained in the agent of the present invention, has a half-life of about 20 hours in human blood plasma. This stability is important, because it is predicted that a peritoneal exudate neutralizes most of protease inhibitors to damage their activity. Aminoalkylphosphonic acids are analogous of alpha-amino acids, and designated by the generally acceptable three-letter abbreviation for an amino acid followed by the superscript P. For example, diphenyl alpha-(N-benzyloxycarbonylamino) ethylphosphonate which is related to alanine is abbreviated Cbz-Ala^{p}(OPh)₂ .

Peptides with a C-terminal phosphate residue which is an analogue of valine, such as Val^{p} (OPh)₂, are relatively effective and relatively specific irreversible inhibitors of elastase and an elastase-like enzyme. The peptides with C-terminal phosphate residue related to phenylalanine, other aromatic amino acids or a long aliphatic acid side chain are relatively effective and relatively effective inhibitors of chymotrypsin and chymotrypsin-like enzyme. The peptides with C-terminal phosphate residue related to ortinine, lysine, arginine or containing a C-terminal diphenyl ester of alpha-amino-alpha-(4-amizinophenyl) methanephosphonate[(4-AmPhGly)^{p}(OPh)₂] or alpha-amino-alpha-(4-amizinophenylmethyl) methanephosphonate [(4-AmPhe)^{p}(OPh)₂] are relatively effective and relatively stable inhibitor of trypsin and trypsin-like enzyme.

Additional specificity and / or increased activation toward reaction with the enzyme can be introduced into the inhibitor molecule by way of variation in the amino acid sequence of the peptide structure site. There is generally a coincidence in sequence between an enzyme substrates such as a peptidyl p-nitroanilides and an effective peptidyl phosphonate inhibitor. Inhibitors with relatively high activity generally have the sequence of a favorable peptidyl p-nitroanilide substrate for a particular enzyme. For example, a relatively potent inhibitor for chymotrypsin and chymotrypsin-like enzymes is Suc-Val-Pro-Phe^{p}(OPh)₂ which has an amino acid sequence that is analogous to Suc-Val-Pro-Phe-NA, a suitable substrate for these enzymes. With human leukocyte elastase, relatively potent inhibitors [Meo-Suc-Ala-Ala-Pro-Val^{p} (OPh)₂ and Boc-Val-Pro-Val^{p} (OPh)₂] have an amino acid sequence similar to MeO-Suc-Ala-Ala-Pro-Val-NA and Boc-Val-Pro-Val-NA, two suitable substrates for this enzyme. It is also possible to design phosphonate inhibitors for serine proteases based on the peptide sequences found in other relatively potent reversible / irreversible inhibitors for those same serine proteases reported in the literature (Powers and Harper, in Protease Inhibitors, Barrett and Salvesen, eds., Elsevier, p. 55-152, 1986; Trainore, D. A. , Trends in Pharm. Sci. 8: 303-307, 1987).

Diphenyl esters of alpha-aminoalkylphosphonate can be synthesized by a previously described method (USP No.5,543,396). Di (substituted phenyl) esters of alpha-aminoalkylphosphonate can be also prepared by the same procedure using tris (substituted phenyl) phosphite instead of triphenyl phosphite. Perfluoroalkyl diesters can be synthesized by a method involving transesterification (Szewczyk et al, Synthesis, 409-414, 1982). Alternatively, the synthesis of diesters of aminoalkylphosphonic acids and their peptides can be performed by esterification of the phosphonic acid moiety as described previously (Bartlett et al., Bioorg. Chem., 14: 356-377, 1986). Numerous additional serine protease inhibitors that may be included in the agent of the present invention have been disclosed in U.S. patents (USP No.6,262,069, No.5,916,888, No.5,900,400, No.5,157,019, No.4,829,052, No.5,723,316, No.5,807,829).

Many organic compounds exist in optically active forms. In describing an optically active compound, the prefixes D and L or R and S are used to denote the absolute configuration of the molecule about its chiral center (s). The prefixes (+) and (-) or d and 1 are employed to designate the sign of rotation of plane-polarized light by the compound. With (-) or 1 meaning that the compound is levorotatory and a compound prefixed with (+) or d is dextrorotatory. For a given chemical structure, these compounds, called stereoisomers, are identical except that they are mirror images of one another. A specific stereoisomer may be also referred to as an enantiomer, and a mixture of such isomers may be called an enantiomeric mixture or racemic. For manufacturing the agent of the present invention, purification of stereoisomer or optical can be utilized as means for greater potency and / or decreased deleterious effects.
As used herein, the term "chiral" refers to molecules which have the property of non-superimposability of the mirror image partner, while the term "achiral" refers to molecules which are superimposable on their mirror image partner. The term "stereoisomers" refers to compound which have identical chemical constitution, but differ with regard to the arrangement of the atoms or groups in space. In particular, "enantiomers" refer to two stereoisomers of a compound, which are non-superimposable mirror images of one another.

"Diastereomers", on the other hand, refers to stereoisomers with two or more centers of asymmetry and whose molecules are not mirror images of one another. With respect to the nomenclature of a chiral center, the terms "S" and "R" configurations are as defined by the IUPAC 1974 (Recommendations for Section E., Fundamental Stereochemistry, Pure Appl. Chem. 45: 13-30 (1976)).

The terms "enantiomerically enriched" and "non-racemic", as used interchangeably herein with reference to compounds used in the agent of the present invention refer to optically enriched compositions, in which one enantiomer is enriched, compared to a racemic mixture of enantiomers in their constituents. Unless otherwise specified, such terms refer to compositons in which the ratio of the desired enantiomer relative to the undesired enantiomer is greater than 1:1. For instance, in enantiomerically enriched preparation, the desirable enantiomer relative to the undesirable enantiomer occupies, at least 50%, more preferably at least 75%, and even more preferably at least 80% by weight. Of course, the enrichment can be much greater than 80% by weight, providing a "substantially enantiomerically enriched", "substantially non-racemic", or "substantially optically pure" preparation, which refers to the agent which contains at least 85% of the desired enantiomer, more preferably at least 90%, and even more preferably at least 95%.

Separation of enantiomers can be accomplished in several ways known in the art. For example, a racemic mixture of two enantiomers can be separated by chromatography ("Chiral Liquid Chromatography", W. J. Lough, Ed. Chapman and Hall, New York (1989)). Enantiomers can also be separated by conventional separation techniques. For example, formation of diastereometric salts and fractional crystallization can be used to separate enantiomers. For the separation of enantiomers of carboxylic acids, the diastereometric salts can be formed by addition of enantiomerically pure chiral bases such as brucine, quinine, ephedrine, and strychnine. Alternatively, diastereometric esters can be formed by separation and hydrolysis of the diastereometric esters to produce enantiomerically enriched carboxylic acids as the free substances, followed by addition of enantiometrically pure chiral alcohols such as menthol. For separation of the optical isomers of amino compounds, addition of chiral carboxylic acid or sulfonic acid such as camphorsulfonic acid, tartaric acid, mandelic acid, or lactic acid can result in formation of the diastereomeric salts. In addition to the separation techniques such as described above, the active enantiomer can be synthesized by stereospecific synthesis to produce only the desired optical isomer using known methodology. Chiral synthesis can produce a product with a high enantiomeric purity. However, in some cases, the enantiomeric purity of the product is not sufficiently high. The skilled artisan will appreciate that the separation methods described above can further enhance the enantiomeric concentration obtained by chiral synthesis. The optical concentration of the enantiomer can be determined by methods known in the art. For example, a sample of enantiomers can be analyzed by high performance liquid chromatography on a chiral chromatographic column.

The chymase inhibitor as a serine protease inhibitor contained in the agent of the present invention is utilized in a method for preventing, inhibiting or treating adhesion formation in the peritoneal cavity of a warm blooded mammal, and can be administered intravenously, orally, or percutaneously to the mammal so that at least one chymase inhibitor may exists by a sufficient amount for a certain period to permit repair of the tissue.

Preferably, the chymase inhibitor which is a peptide derivative of the aryl diester of alpha-aminoalkylphosphonic acid such as Suc-Val-Pro-Phe^{p} (OPh)₂ can be utilized. More preferably, the preparation of a peptide derivative of the aryl diester of alpha-aminoalkylphosphnaic acid such as Suc-Val-Pro-L-Phe^{p} (OPh)₂ which has been enantiomerically enriched can be utilized. This Suc-Val-Pro-L-Phe^{p} (OPh)₂, comprises greater than 50%, preferably 80%, or more preferably 95% or more by weight of the total Suc-Val-Pro-Phe^{p} (OPh)₂. The warm blooded mammal is desirably human. In order to enrich, for example, acetone-ether is used to crystallize repeatedly.

### (Preparation)

The agent of the present invention may be prepared and administered as a pharmaceutical composition comprising the above-described protease inhibitor alone or in combination with a suitable additive. The pharmaceutical composition is not particularly limited, as long as it is used for oral or non-oral administration. For example, tablet, syrup, ample for injection, freeze dried powder for injection, ointment, wet pack may be utilized. Various types of preparations can be made in accordance with the conventional method using a known preparation additives available for those skilled in the art such as diluent, additives.

For example, the freeze-dried powder for injection, can be prepared by dissolving an effective amount of the purified protease inhibitor in a dilution solution such as a distilled water for injection, a physiologic saline, or an aqueous glucose solution, and adding, if necessary, an excipient such as carboxylmethylcepharose, or sodium alginic acid, a stabilizing agent such as polyethylene glycol, sodium dextran sulfate, an amino acid, or human serum albumin, a conservation agent such as benzyl alcohol, benzalkonium chloride, or phenol, a soothing agent such as glucose, calcium gluconate, or procaine hydrochloride, and a pH adjusting agent such as hydrochloric acid, acetic acid, citric acid, or sodium hydroxide to make by a conventional method. Moreover, the ampoule preparation for injection can be prepared by dissolving an effective amount of the protease inhibitor in a dilution solution such as a distilled water for injection, a physiologic saline, or Ringer's solution, and adding, if necessary, an auxiliary dissolving agent such as sodium salicylate, or mannitol, a buffer such as sodium citrate, or glycerin, an isotonic agent such as glucose or invert sugar, an additive such as a stabilizing agent, a conservation agent, a soothing agent, or a pH adjusting agent, followed by sterilizing such as a conventional heat sterilization, or filtration sterilization. The sterilization method should be appropriately selected because heat sterilization process can inactivate depending on the kind of the active component.

The agent of the present invention can be made a dosage form suitable for oral or non-oral administration in a solid state such as a tablet, a granule, a capsule, or a powder, or in a liquid state such as a solution, a suspension, a syrup, an emulsion, or a lemonade, an ointment, a wet pack using a pharmaceutically acceptable carrier. If necessary, an auxiliary agent, a stabilizing agent, a moistening agent, and the other conventional additive such as lactic acid, citric acid, tartaric acid, stearic acid, magnesium stearate, white clay, sucrose, corn starch, tarc, gelatin, agar, pectin, peanuts oil, olive oil, cacao oil, ethylene glycol may be blended in the above-described preparation.

### (Dose)

The agent of the present invention contains an effective amount of at least one protease inhibitor, and can be administered so that the protease inhibitor may be substantially retained at an effective concentration for a sufficient time to permit epithelialization in the site of adhesion formation.

In the present invention, the acceptable amount and concentration of a protease inhibitor to administer can be determined within the range from the lowest concentration for achieving the effect, that is, "an effective amount", to the highest concentration, "a pharmaceutically acceptable", "a pharmacologically acceptable" concentration. A mixture of protease inhibitors can be administered intravenously or orally in a dosage form or medium (physiological saline) which is so suitable that the desired effect may be obtained in a site (abdominal, thoracic, ophthalmic, cardiac, gynecologic tissue and the like) to prevent, inhibit or treat the adhesion formation.

In the present invention, the term "effective amount" means a sufficient amount of the agent for gaining a desired reaction with little or no toxicity to prevent, inhibit or treat the adhesion formation. The precise required amount varies depending on an individual subject in species, age, body weight, general body conditions, or administration mode. A suitable "effective amount" can be also determined by the general prior art using fact provided herein and conventional methods.

The term "pharmaceutically acceptable" refers, in consideration of effect versus danger in ratio, to a substance, a compound, a mixture or an administration mode which is appropriate to contact with human or animal tissue within a reliable medical discretion and free from accompanying excessive toxicity, inflammation, allergic response, the other troubles or complications.

The relevant protease inhibition compound can be administered at an interval for generally acting, for example, before, during, or after the operation. The administration can be initiated at least between 1 hour and 72 hours, and preferably between 1 hour and 48 hours after the operation. For example, in the case where it is intravenously administered, the administration can be initiated between 1 hour and 24 hours, and preferably between 1 hour and 12 hours after the operation, and in the case where it is orally administered, the administration can be initiated similarly, that is, between 1 hour and 72 hours, preferably between 12 hours and 48 hours, and more preferably between 12 hours and 24 hours.

The agent of the present invention allows at least one protease inhibitor to be administered in a substantially sufficient amount so that the inhibitor may exist by an effective concentration in the site of heart to protect. For example, chymase inhibitor (Suc-Val-Pro-L-Phe^{p}(Oph)₂) which is a specific example of protease inhibitor contained in the medication of the present invention, could be used at 10 *µ*M during the operation, thereby to suppress significantly the chymase activity in the blood vessel tissue of the living body for 4 weeks. The effective administration amount of chymase inhibitor (Suc-Val-Pro-L-Phe^{p} (Oph)₂) can be selected in the range from 0.0001 to 100 mg per 1 kg of adult body weight, per one day. For example, it is administered intravenously by an amount selected in the range from 0.001 to 100 mg, preferably from 0.01 to 1 mg, and orally by an amount selected in the range from 0.1 to 100 mg, preferably from 0.1 to 10 mg.

An effective amount and pathway for the other protease inhibitors can be determined by known means.

### (Examples)

The present invention will be concretely described by Examples below, but is not limited to them.

### (Example 1)

### Preparation of aminoalkylphosphonic acid derivatives

Agents comprising peptidyl derivatives of aryl diesters of aminoalkylphosphonic acid have been demonstrated and performed by one of the prior art (Biochemistry, 30, p.485-493, 1991).

To elucidate the relationship between chymase activity and adhesion formation, the effect of a representative serine protease inhibitor, the chymase inhibitor, Suc-Val-Pro-Phe^{p}(OPh)₂, was tested. This compound has been synthesized using a known methodology (Biochemistry, 30, p.485-493, 1991). More specifically, the reaction of Cbz-Val-OH (0.25 g, 1 mmol), DCC (0.2 g, 1 mmol) and the product of hydrogenolysis of Cbz-Val-Pro-Phe^{p} (OPh)₂ (0.584 g, 1 mmol) were dissolved in 30 ml of ethyl acetate and oil was added thereto. To this solution, 0.1 g (1 mmol) of succinic anhydride and 0.1 g of 5% Pd/C were added and the mixture was stirred under a hydrogen atmosphere until thin layer chromatography (ILC) showed only one new spot. The catalyst was removed by filtration and the organic layer was washed several times with water. After drying, the organic solvent was removed to give, for example, 0.45 g (65%) of a product as a hydroscopic solid (mp.50-53°C: one apot on TLC, R_{f}=0.4; ³¹P NMR 19.75, 19.23 ppm, ratio 1:1, Anal. Calcd. for C₃₄H₄₀O₃N₃P.2H₂O: 59.56; H, 6.42. Found: C, 59.59; H, 6.42) .

### (Example 2)

### Preparation of enriched enantiomers of chymase inhibitor Suc-Val-Pro-Phe^{p} (OPh)₂

As used below, the following abbreviations apply: Z is Benzyloxycarbonyl, Boc is tert-butyloxycarbonyl, WSCD is carbodiimide, and HOBt is 1-hydroxybenzotriazol. Z-Phe^{p} (OPh)₂ was synthesized according to the procedure of Oleksyszyn and Powers (Methods Enzymol. 244: 423-441, 1994) and benzyloxycarbonyl was removed by hydrogen bromide/acetic acid solution. The product was coupled with Boc-Pro by WSCD-HOBt reaction and then the racemic mixture was obtained. The racemic mixture then was separated by re-precipitation. Inactive enantiomer was firstly crystallized from the solution and removed. After de-blocking of Boc with HCl from the active enantiomer in the solution, the sample was coupled with Boc-Val by WSCD-HOBt reaction and deblocked again to separate. To this product, succinic anhydride and triethyamine were added to get Suc-Val-Pro-Phe^{p} (OPh)₂. The product was finally enriched by reverse phase HPLC.

### (Z-DL-Phe^{p} (OPh)₂)

Phenylacetaldehyde (28.3 mL, 0.242 mol) was dissolved in 45 mL of acetic acid. Carbamic acid benzyl ester (24.4 g, 0.161 mol) and triphenyl phosphite (50.0 g, 0.161 mol) were added to this solution, followed by stirring at 85°C for 1.5 h. After the organic solvent was evaporated, the residual solution was cooled to room temperature, supplied with 400 mL of methanol, and allowed to crystallize at -20°C. The product was filtered to collect, washed with cold methanol and dried *in vacuo* to yield 32.9 g (42%) of compounds of Z-D-Phe^{p}(OPh)₂ and Z-L-Phe^{p} (OPh)₂.

### (DL-Phe^{p} (OPh) · HBr)

The mixture of Z-D-Phe^{p} (OPh)₂ and Z-L-Phe^{p} (OPh) ₂ (14.3 g, 29.3 mmol) was dissolved in 30 mL of 25% hydrogen bromide/acetic acid solution and stirred at room temperature for 1 hour. A solid sample separated after addition of the both was filtered to collect, washed with ether and dried *in vacuo* to yield 12 g (94%) of a mixture of D and L-Phe^{p}(OPh)₂ HBr.

### (Boc-Pro-DL-Phe^{p} (OPh)₂)

A mixture of D and L-Phe^{p} (OPh)₂ HBr (11.5 g, 26 mmol), Boc-Pro (5.53 g, 25.7 mmol) and HOBt (3.58 g, 26.5 mmol) was dissolved in 70 mL of DMF, and then WSCD (4.70 mL, 26.5 mmol) was added dropwise thereto under cooling with ice. After stirring at room temperature for 3.5 hours, the solution was concentrated *in vacuo,* and ethyl acetate was added. The resultant solution was successively washed with acetic acid and an alkaline solution, dried over MgSO₄, filtered and concentrated *in vacuo.* The crystallization from acetone-ether was repeated to remove the inactive enantiomer (D) and obtain Boc-Pro-L-Phe^{p}(OPh)_{2.} The residual solution was concentrated *in vacuo* and purified by medium-pressure silica-gel chromatography using toluene-ethyl acetate (5:1) as an eluent to yield 5.10 g of the enantiomer.

### (Boc-Pro-L-Phe^{p} (OPh)₂)

Boc-Pro-L-Phe^{p} (OPh)₂ (4,98 g, 9.05 mmol) was dissolved in 37 mL of cold HCl / dioxane (4.9 N), followed by stirring at room temperature for one hour. The solution was evaporated and dried in vacuo. The residue was dissolved in 40 mL of DMF, and then Boc-Val (2.06 g, 9.50 mmol) and HOBt (1.35 g, 9.96 mmol) were added to this solution. WSCD (1.77 mL, 9.96 mmol) was added dropwise into this solution under cooling with ice, followed by stirring at room temperatuture over night. This reaction solution was supplied with Ethyl acetate, successively washed with an acidic solution and an alkaline solution, dried over MgSO₄ and filtered to yield 6.26 g (94%) of a colorless oil, which was concentrated *in vacuo.*

### (Suc-Pro-L-Phe^{p} (OPh)₂)

Boc-Pro-L-Phe^{p} (OPh)₂ (5.86 g, 8.47 mmol) was dissolved in 35 mL of cold HCl / dioxane (4.9 N), followed by stirring at room temperature for one hour. The solution was evaporated and dried in vacuo. The residue was dissolved in 35 mL of DMF, and then succinic anhydride (1. 02 g, 10.2 mmol) was added thereto. Triethylamine (2.36 mL, 16.9 mmol) was added dropwise into this solution, followed by stirring at room temperature for two hours. Under cooling with ice, the solution was adjusted by HCl to have a pH of 1.0, and ethyl acetate was added thereto to extract. The extract was washed with a saturated saline solution, dried over MgSO₄, and filtered to give an yellow oil, which was filtered and concentrated *in vacuo.* The resulting oil was enriched by the reverse-phase HPLC (Column: YMC ODS SH-363-5, 30 x 250 mm, Mobile phase: 0.1 TFA gradient 40-70% MeCN) to yield 2.30 g (42%) of a white powder. The powder was cooled to dry.

### (Results of analysis)

Sample: Suc-Val-Pro-L-Phe^{p} (OPh)₂
Lot No.: 520217
Volume: 2.0 g x 1
Appearance: White powder
Purity (by HPLC): Main peak 98.8%
HPLC condition
Column: YMC Pack, ODS-A 4.6mm I.D. x 150 mm
Mobile phase: 0.1 % TFA, gradient 30-80% MeCN (25 min)
Flow rate: 1.0 mL/min
Detector: An ultraviolet absorption photometer (wavelength: 220 nm)

| Amino acid analysis: | molar ratio | recovery |
|---|---|---|
| Val (1) | 1.00 | 90.5% |
| Pro (1) | 1.03 | |
| Phe^{p} (OH)₂ | 0.98 | |

Hydrolysis condition: 6 mol HCl, phenol, 110°C, 22 hours
Elemental analysis: Calcd for C₃₄H₄₀N₃O₈S : C, 62.86: H, 6.21; N, 6.47%
Calcd for C₃₄H₄₀N₃O₈S 8H₂O-0.5TFA: C, 58.30; H, 5.88; N, 5.83%
Hound: C, 58.34; H, 5.94; N, 5.51%
Mass spectrometry: 650.3 (Calcd [M+H]_{exact}=650.253 (by ESI-MS))

### (Experimental Example) Survival rate after the occurrence of myocardial infarction resulted by oral administration of Suc-Val-Pro-L-Phe^{p}(OPh)₂

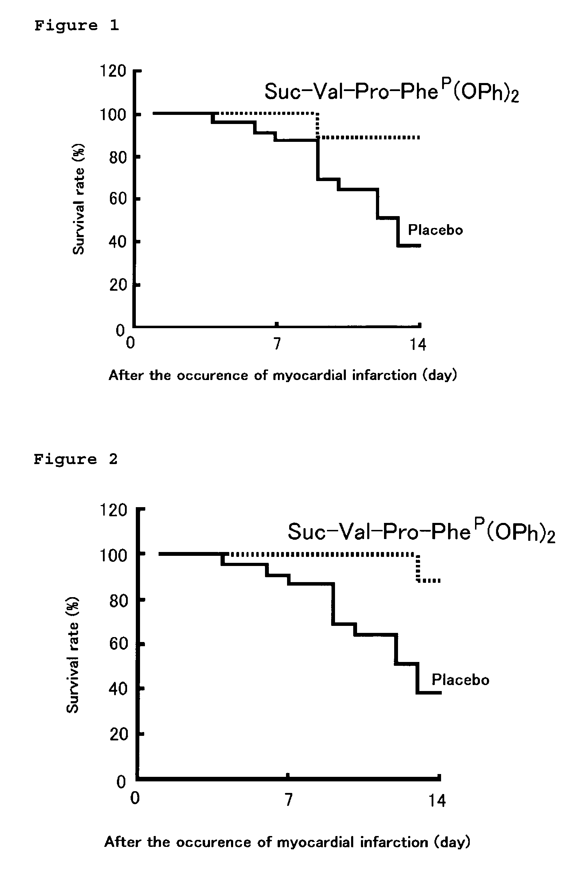

The left coronary artery was ligated in Syrian hamsters (SLC, Co., Ltd., Shizuoka, Japan), 6 weeks of age, weighing 85 to 90 g, to prepare a myocardial infarction model (Jpn.J.Pharmacol., 86, p. 203-214 2001 life Sci. 71 p. 437-446 2002). Suc-Val-Pro-L-Phe^{p} (OPh)₂ (10 mg/kg) (nine cases) or a placebo (twenty three cases) has been forcibly orally administered using a sound once a day from the 3rd day before the model preparation to the 14th day after the model preparation, and the degree of adhesion up to the 14th day following the model preparation was compared to study. It should be noted that the significant test was performed by log rank test using the survival curve.
As a result, the survival rate of the placebo group up to the 14th day was 39.1%, while that of the group in which Suc-Val-Pro-L-Phe^{p} (OPh)₂ (10 mg/kg) was administered from the day before the operation was 88 . 9%, indicating that the survival rate was significantly increased in the Suc-Val-Pro-L-Phe^{p}(OPh)₂ administration group (see Fig.1).
Similarly, the survival rate of the placebo group up to the 14th day was 39.1%, while that of the group in which Suc-Val-Pro-L-Phe^{p}(OPh)₂ (10 mg/kg) was administered orally from the day after the operation was 88.9%, indicating that the survival rate was significantly increased in the Suc-Val-Pro-L-Phe^{p} (OPh)₂ administration group (see Fig.2).
Suc-Val-Pro-L-Phe^{p} (OPh)₂ was administered before occurrence of myocardial infarction, allowing significant improvement of the survival rate, and further administered even after occurrence of myocardial infarction, allowing significant improvement. This indicates that Suc-Val-Pro-L-Phe^{p}(OPh)₂ is useful for preventing myocardial infarction and for improving the survival rate after myocardial infarction occurs.

### [Industrial Applicability]

As described above, the agent for protecting cardiac damage of the present invention is administered orally, allowing prevention of myocardial infarction and improvement of the survival rate even after the occurrence of myocardial infarction. Therefore, it is suggested that the agent is useful as an agent for protecting cardiac damage against arrhythmia, cardiac desmoplasia and heart-failure accompanying with myocardial infarction or the like.

### [Brief Description of the Drawings]

Fig. 1 is a graph showing the survival rate when a placebo or Suc-Val-Pro-L-Phe^{p}(OPh)₂ was administered orally from the day before the occurrence of myocardial infarction (Experimental Example); and,
Fig. 2 is a graph showing the survival rate when a placebo or Suc-Val-Pro-L-Phe^{p} (OPh)₂ was administered orally from the day after the occurrence of myocardial infarction (Experimental Example).

## Claims

1. An agent for protecting cardiac damage, wherein the agent contains an effective amount of at least one protease inhibitor and is administered intravenously or orally.

2. The agent for protecting cardiac damage according to Claim 1, wherein the protease inhibitor is a serine protease inhibitor.

3. The agent for protecting cardiac damage according to Claim 2, wherein the serine protease inhibitor is a chymotrypsin-like serine protease inhibitor.

4. The agent for protecting cardiac damage according to Claim 3, wherein the chymotrypsin-like serine protease inhibitor is a chymase inhibitor.

5. The agent for protecting cardiac damage according to Claim 4, wherein the chymase inhibitor is a peptide derivative of aryl diester of alpha-aminoalkylphosphonic acid.

6. The agent for protecting cardiac damage according to Claim 4, wherein the chymase inhibitor is Suc-Val-Pro-Phe^{p} (OPh)₂.

7. The agent for protecting cardiac damage according to Claim 4, wherein the chymase inhibitor is an enriched preparation of enantiomer Suc-Val-Pro-L-Phe^{p} (OPh)₂ of Suc-Val-Pro-Phe^{p} (OPh)₂.

8. The agent for protecting cardiac damage according to Claim 7, wherein the Suc-Val-Pro-L-Phe^{p}(OPh)₂ comprises greater than 95% by weight of the total Suc-Val-Pro-Phe^{p}(OPh)₂ in the enriched preparation of the enantiomer.

9. The agent for protecting cardiac damage according to any one of Claims 1-8, wherein the protease inhibitor is bound to a transmitter for maintaining an effective local concentration of the protease inhibitor in the relevant site and then administered, the transmitter being a carrier having a high molecular weight selected from the group consisting of hyaluronic acid, hydrogel, carboxymethylcellose, dextran, cyclodextran and a composition of compounds thereof.

10. An agent mixture for protecting cardiac damage, comprising the protease inhibitor according to any one of Claims 1-9 and a pharmaceutically acceptable diluent solution or excipient.

11. A method for improving arrhythmia, cardiac desmoplasia and / or heart-failure, wherein the agent mixture for protecting cardiac damage according to Claim 10 is administered to a vertebrate subject in a case where the arrhythmia, cardiac desmoplasia, and heart-failure are likely to accompany with hypertension, hypercardia, myocardial infarction, arteriosclerosis, diabetic and non-diabetic renal diseases, re-stenosis posterior to PTCA.

12. A use of the agent mixture for protecting cardiac damage according to Claim 10, wherein the use comprises making a medicine which is applied against arrhythmia, cardiac desmoplasia and / or heart-failure in a case where the arrhythmia, cardiac desmoplasia, and heart- failure are likely to accompany with hypertension, hypercardia, myocardial infarction, arteriosclerosis, diabetic and non-diabetic renal diseases, and re-stenosis posterior to PTCA.

13. A use of the agent mixture for protecting cardiac damage according to Claim 10, wherein the use comprises using as a agent for improving arrhythmia, cardiac desmoplasia and / or heart- failure in a case where the arrhythmia, cardiac desmoplasia, and heart-failure are likely to accompany with hypertension, hypercardia, myocardial infarction, arteriosclerosis, diabetic and non-diabetic renal diseases, and re-stenosis posterior to PTCA.
